# EUROPEAN PATENT SPECIFICATION

(11) **EP 1 853 606 B1**
(45) Date of publication and mention of the grant of the patent: **19.11.2008**
(21) Application number: 06720539.3
(22) Date of filing: 09.02.2006
(51) Int. Cl.: C07D 471/14, A61K 31/495, A61P 35/00, A61P 3/10

(54) **PYRIDOPYRAZOLOPYRIMIDINE COMPOUNDS AND THEIR USES AS ANTI-CANCER AND ANTI-DIABETE DRUGS**
PYRIDOPYRAZOLOPYRIMIDINDERIVATE UND IHRE ANWENDUNG GEGEN KREBS UND DIABETES
DERIVES PYRIDOPYRAZOLOPYRIMIDINIQUES ET LEUR UTILISATION COMME AGENTS ANTICANCEREUX ET ANTIDIABETIQUES

(30) Priority: 10.02.2005 US 651827 P
(43) Date of publication of application: 14.11.2007
(73) Proprietor: SMITHKLINE BEECHAM CORPORATION, Philadelphia, PA 19101 (US)
(72) Inventor: JUNG, David K., GlaxoSmithKline, Corp. Intellect. Property Dept., Research Triangle Park, NC 27709 (US); ALBERTI, Michael J., GlaxoSmithKline, Corp. Intellectual Property Dept., Research Triangle Park, NC 27709 (US)
(74) Representative: Priestley, Ruth Elizabeth
(86) International application number: PCT/US2006/004547
(87) International publication number: WO 2006/086539

(56) References cited:
- ARQUES, A. ET AL: "The reaction of 1-amino-4,6-diphenyl-2-methylthiopyridiniu m iodide with substituted acetonitriles" SYNTHESIS , (11), 910-12 CODEN: SYNTBF; ISSN: 0039-7881, 1981, XP002384880
- BHAT ET AL: "Pyrazolopyrimidine Nucleosides. 12. Synthesis and Biological Activity of Certain Pyrazolo[3,4-d]pyrimidine Nucleosides Related to Adenosine" JOURNAL OF MEDICINAL CHEMISTRY, AMERICAN CHEMICAL SOCIETY. WASHINGTON, US, vol. 24, 1981, pages 1165-1172, XP002267111 ISSN: 0022-2623
- ALBERTI, MICHAEL J. ET AL: "Discovery and in vitro evaluation of potent kinase inhibitors: Pyrido[1',2':1,5]pyrazolo[3,4-d]pyrimidine s" BIOORGANIC & MEDICINAL CHEMISTRY LETTERS , 15(16), 3778-3781 CODEN: BMCLE8; ISSN: 0960-894X, 2005, XP002384881

## Description

### FIELD OF THE INVENTION

The present invention relates to pyridopyrazolopyrimidine derivatives that are useful pharmacological agents for disease states that are mediated, for example alleviated, through the inhibition or antagonism, of protein kinases, and to processes for the preparation and use of the same. In particular, the present invention relates to compounds that demonstrate protein tyrosine kinase and/or protein serine/threonine kinase inhibition.

### BACKGROUND OF THE INVENTION

The protein kinases represent a large family of proteins that play a central role in regulating a wide variety of cellular processes and maintaining control over cellular function (Hanks, et al., Science, 1988, 241, 42-52). The loss of control over cellular regulation frequently leads to aberrant cell function or death, often leading to disease in the parent organism.

Inhibiters of specific kinases are useful in a variety of therapies. Examples of kinase inhibitors used in cancer therapy include c-Src (Brickell, Critical Reviews in Oncogenesis 1992, 3, 401-46; Courtneidge, Seminars in Cancer Biology 1994, 5, 239-46), raf (Powis, Pharmacology & Therapeutics 1994, 62, 57-95) and the cyclin-dependent kinases (CDKs) 1, 2 and 4 (Pines, Current Opinion in Cell Biology 1992, 4, 144-8; Lees, Current Opinion in Cell Biology 1995, 7, 773-80; Hunter and Pines, Cell 1994, 79, 573-82). Other therapies include inhibition of kinases such as CDK5 and GSK3 for Alzheimer's disease (Hosoi, et al., Journal of Biochemistry (Tokyo) 1995, 117, 741-9; Aplin, et al., Journal of Neurochemistry 1996, 67, 699-707), inhibition of c-Src kinase for osteoporosis (Tanaka, et al., Nature 1996, 383, 528-31), inhibition of GSK-3 kinase in type-2 diabetes (Borthwick, et al., Biochemical & Biophysical Research Communications 1995, 210, 738-45), and inhibition of the p38 kinase for inflammation (Badger, et al., The Journal of Pharmacology and Experimental Therapeutics 1996, 279, 1453-61). Also, inhibitors of VEGF-R 1-3 and TIE-1 and -2 kinases are useful in treating diseases that involve angiogenesis (Shawver, et al., Drug Discovery Today 1997, 2, 50-63).

As noted above, GSK3 (glycogen synthase kinase) is a kinase useful in the treatment of type II diabetes. GSK3 inhibits glycogen synthase by direct phosphorylation. Upon insulin activation, GSK3 is inactivated, thereby allowing the activation of glycogen synthase and possibly other insulin-dependent events.

Type II diabetes, otherwise known as non-insulin dependent diabetes mellitus (NIDDM), is initially characterized by decreased sensitivity to insulin (insulin resistance) and a compensatory elevation in circulating insulin concentrations. Increased insulin levels are caused by increased secretion from the pancreatic beta cells in an attempt to overcome the insulin resistance. The resulting hyperinsulinemia is associated with a variety of cardiovascular complications.

As insulin resistance worsens, the demand on the pancreatic beta cells steadily increases until the pancreas can no longer provide adequate levels of insulin, thereby resulting in elevated levels of glucose in the blood. Thus, diabetes causes impaired glucose transport into skeletal muscle and increased hepatic glucose production, in addition to inadequate insulin response. The disorders and conditions associated with hyperglycemia and hyperlipedemia include cardiovascular disease, renal failure, and blindness.

GSK3 inhibition stimulates insulin-dependent processes and is consequently useful in the treatment of diseases and conditions, such as type II diabetes, that are mediated by GSK3 activity, or, more specifically, characterized by a need for the inhibition of GSK3. For example, Klein et al., PNAS 93:8455-9 (1996) report that lithium ion inhibits GSK3 activity. Lithium has been reported to have anti-diabetic effects such as reduction of plasma glucose levels, increased glycogen uptake, potentiation of insulin, and stimulation of glycogen synthesis in skin, muscle, and fat cells. Lithium, however, effects molecular targets other than GSK3, and is, therefore, not a widely accepted therapy for diabetics.

GSK3 is a proline-directed serine/threonine kinase. Other examples of GSK3 mediated diseases or conditions include obesity, various CNS disorders such as Alzheimer's Disease, bipolar disorder, and schizophrenia, neurotraumatic injuries such as acute stroke, immune potentiation, baldness or hair loss, atherosclerotic cardiovascular disease, hypertension, polycystic ovary syndrome, ischemia, brain trauma or injury, immunodeficiency, and cancer. See, for example, published PCT application WO 00/38675.

In cancer the growth of solid tumors has been shown to be dependent on angiogenesis. The progression of leukemias as well as the accumulation of fluid associated with malignant ascites and pleural effusions also involve pro-angiogenic factors. (See Folkmann, J., J. Nat'l. Cancer Inst., 1990, 82, 4-6.) Consequently, the targeting of pro-angiogenic pathways is a strategy being widely pursued in order to provide new therapeutics in these areas of great, unmet medical need.

Central to the process of angiogenesis are vascular endothelial growth factor (VEGF) and its receptors, termed vascular endothelial growth factor receptor(s) (VEGFRs). Three protein tyrosine kinase receptors for VEGF have been identified: VEGFR1 (Flt-1); VEGFR2 (Flk-1 and KDR) and VEGFR3 (Flt-4). These receptors are involved in angiogenesis and participate in signal transduction. (Mustonen, T. et al J. Cell Biol. 1995:129:895-898; Ferrara and Davis-Smyth, Endocrine Reviews, 18(1):4-25, 1997; McMahon, G., The Oncologist, Vol. 5, No 90001, 3-10, April 2000).

Of particular interest is VEGFR-2, which is a transmembrane receptor protein tyrosine kinase expressed primarily in endothelial cells. Activation of VEGFR-2 by VEGF is a critical step in the signal transduction pathway that initiates tumor angiogenesis. Consequently, antagonism of the VEGFR-2 kinase domain would block phosphorylation of tyrosine residues and serve to disrupt initiation of angiogenesis, thereby providing a potent treatment for cancer or other disorders associated with inappropriate angiogenesis.

The erbB family of protein tyrosine kinases is another group of such kinases that are implicated in human malignancies. Elevated Erb B1 receptor activity has, for example, been implicated in non-small cell lung, bladder, renal cell, and head and neck cancers. Increased c-erbB-2 activity is associated with breast, ovarian, gastric and pancreatic cancers. Consequently, inhibition of such protein tyrosine kinases should provide a treatment for disorders characterized by aberrant Erb family protein kinase activity.

Thus, the compounds of the present invention are believed useful is a variety of disease states, each of which may be characterized as mediated by inhibition or antagonism of protein kinases.

### SUMMARY OF THE INVENTION

Briefly, in one aspect, the present invention provides compounds of formula (I) 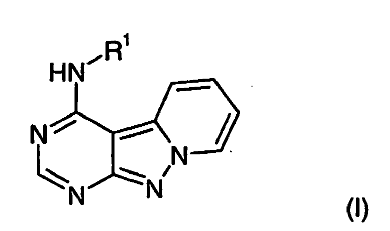 or a salt or solvate thereof, wherein
R¹ is H, substituted aryl, substituted heteroaryl, -C(O)N(H)R², or -N=CR³, wherein when R¹ is substituted aryl or substituted heteroaryl, each substituent is independently selected from the group consisting of aryl, halo, or -OR⁴;
R² is substituted aryl, wherein each substituent is independently selected from the group consisting of cyano, halo, nitro, or haloalkyl;
R³ is heteroaryl; and
R⁴ is aryl or haloaryl.

Another aspect of the present invention provides a pharmaceutical composition comprising a compound of formula (I).

Another aspect of the present invention provides the administration of a compound of formula (I) for the use in the treatment of cancer or diabetes.

Another aspect of the present invention provides a compound of formula (I) for the use in therapy, and in particular, in human medicine.

Another aspect of the present invention provides the use of a compound of formula (I) for the manufacture of a medicament for the treatment of cancer or diabetes.

### DETAILED DESCRIPTION OF THE INVENTION

Terms are used within their accepted meanings. The following definitions are meant to clarify, but not limit, the terms defined.

As used herein, "a compound of formula (I)" means a compound of formula (I), or a salt or solvate thereof.

As used herein the term "alkyl" refers to a straight or branched chain hydrocarbon, preferably having from one to twelve carbon atoms. Examples of "alkyl" as used herein include, but are not limited to, methyl, ethyl, propyl, isopropyl, isobutyl, n-butyl, tert-butyl, isopentyl, and n-pentyl.

As used herein, the term "aryl" refers to an aromatic ring system, such as a benzene ring system, such as phenyl. The term encompasses fused systems where one or more benzene rings form, for example, anthracene, phenanthrene, or naphthalene ring systems. The term also includes an optional alkylene linker, such as C1-C6 alkylene, through which the aryl group may be attached. Examples of "aryl" groups include, but are not limited to phenyl, benzyl, 2-naphthyl, 1-naphthyl, and biphenyl.

As used herein, the term "heteroaryl" refers to a monocyclic five to seven membered aromatic ring, or to a fused bicyclic aromatic ring system comprising two of such aromatic rings, which contain one or more nitrogen, sulfur, and/or oxygen atoms, where N-oxides, sulfur oxides, and dioxides are permissible heteroatom substitutions. Examples of "heteroaryl" groups used herein include, but should not be limited to, furan, thiophene, pyrrole, imidazole, pyrazole, triazole, tetrazole, thiazole, oxazole, isoxazole, oxadiazole, thiadiazole, isothiazole, pyridine, pyridazine, pyrazine, pyrimidine, quinoline, isoquinoline, benzofuran, benzothiophene, benzimidazole, indole, indazole, and the like. Preferred heteroaryl groups include benzimidazolyl and indolyl.

As used herein the term "halo" refers to -F, -Cl, -Br, or -I.

As used herein the term "haloalkyl" refers to an alkyl group, as defined herein that is substituted with at least one halogen. Examples of branched or straight chained "haloalkyl" groups useful in the present invention include, but are not limited to, methyl, ethyl, propyl, isopropyl, n-butyl, and t-butyl substituted independently with one or more halogens, e.g., fluoro, chloro, bromo, and iodo. The term "haloalkyl" should be interpreted to include such substituents such as -CF₃, -CH₂-CH₂-F, -CH₂-CF₃, and the like.

As used herein the term "haloaryl" refers to an aryl group, as defined herein that is substituted with at least one halogen. Examples of "haloaryl" groups useful in the present invention include, but are not limited to, phenyl and naphthyl, substituted independently with one or more halogens, e.g., fluoro, chloro, bromo, and iodo.

As used herein the term "nitro" refers to a group -NO₂.

As used herein the term "cyano" refers to a group -CN.

As used herein, the term "solvate" refers to a complex of variable stoichiometry formed by a solute (in this invention, a compound of formula I or a salt thereof) and a solvent. Such solvents for the purpose of the invention may not interfere with the biological activity of the solute. Examples of suitable solvents include, but are not limited to, water, methanol, ethanol and acetic acid. Preferably the solvent used is a pharmaceutically acceptable solvent. Examples of suitable pharmaceutically acceptable solvents include water, ethanol and acetic acid. Most preferably the solvent used is water.

Typically, the salts of the present invention are pharmaceutically acceptable salts. Salts encompassed within the term "pharmaceutically acceptable salts" refer to non-toxic salts of the compounds of this invention. Salts of the compounds of the present invention may comprise acid addition salts derived from a nitrogen on a substituent in a compound of the present invention. Representative salts include the following salts: acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, laurate, malate, maleate, mandelate, mesylate, methylbromide, methylnitrate, methylsulfate, monopotassium maleate, mucate, napsylate, nitrate, N-methylglucamine, oxalate, pamoate (embonate), palmitate, pantothenate, phosphate/diphosphate, polygalacturonate, potassium, salicylate, sodium, stearate, subacetate, succinate, tannate, tartrate, teoclate, tosylate, triethiodide, trimethylammonium and valerate. Other salts, which are not pharmaceutically acceptable, may be useful in the preparation of compounds of this invention and these form a further aspect of the invention.

A preferred aspect of the present invention is a compound of formula (I) wherein R¹ is H.

Another preferred aspect of the present invention is a compound of formula (I) wherein R¹ is substituted aryl, or substituted heteroaryl, wherein each substituent is independently selected from the group consisting of aryl, halo, and -OR⁴, and R⁴ is aryl or haloaryl.

Another preferred aspect of the present invention is a compound of formula (I) wherein R¹ is -C(O)N(H)R², and R² is substituted aryl, wherein each substituent is independently selected from the group consisting of cyano, halo, nitro, and haloalkyl.

A further aspect of the present invention is a compound of formula (I), wherein said compound is selected from: 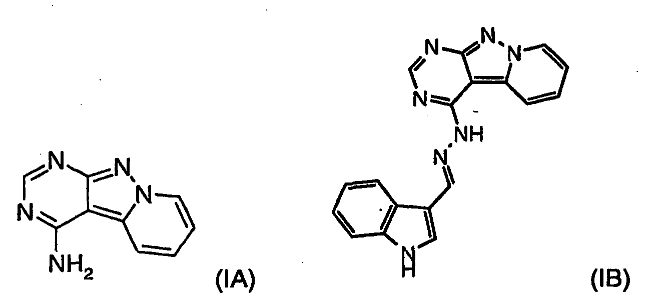 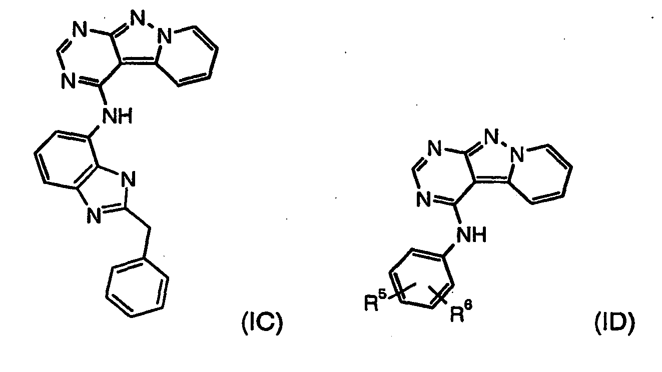 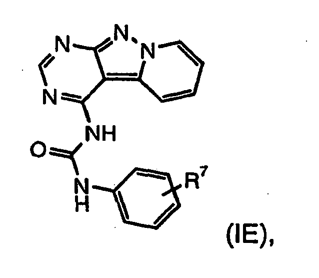 and salts and solvates thereof, wherein R⁵ and R⁶ are independently selected from the group consisting of hydrogen, halo, and -OR⁴, R⁴ is aryl or haloaryl, and R⁷ is cyano, halo, nitro, and haloalkyl.

While the preferred groups for each variable have generally been listed above separately for each variable, preferred compounds of this invention include those in which several or each variable of formula (I) is selected from the preferred, more preferred, or most preferred groups for each variable. Therefore, this invention is intended to include all combinations of preferred, more preferred, and most preferred groups.

Specific compounds of formula (I) include but are not limited to those compounds described in the Example section that follows. Suitable compounds of the present invention include:
pyrido[1',2':1,5]pyrazolo[3,4-*d*]pyrimidin-4-amine;
*N*-(3-chloro-4-fluorophenyl)pyrido[1',2':1,5]pyrazolo[3,4-*d*]pyrimidin-4-amine;
*N*-(3-chloro-4-{[(3-fluorophenyl)methyl]oxy}phenyl)-pyrido[1',2':1,5] pyrazolo[3,4-*d*]pyrimidin-4-amine;
*N*-{3-chloro-4-[(phenylmethyl)oxy]phenyl}pyrido[1',2':1,5]pyrazolo[3,4-*d*]pyrimidin-4-amine;
*N*-[2-(phenylmethyl)-1*H*-benzimidazol-5-yl]pyrido[1',2':1,5]pyrazolo-[3,4-*d*]pyrimidin-4-amine;
*N*-(3-cyanophenyl)-*N*'-pyrido[1',2':1,5]pyrazolo[3,4-*d*]pyrimidin-4-ylurea;
*N*-pyrido[1',2':1,5]pyrazolo[3,4-*d*]pyrimidin-4-yl-*N*'-[3-(trifluoromethyl)phenyl]urea;
*N*-(3-nitrophenyl)-*N*'-pyrido[1',2':1,5]pyrazolo[3,4-*d*]pyrimidin-4-ylurea;
*N*-(4-bromophenyl)-*N*'-pyrido[1',2':1,5]pyrazolo[3,4-*d*]pyrimidin-4-ylurea;
*N*-pyrido[1',2':1,5]pyrazolo[3,4-*d*]pyrimidin-4-yl-*N*'-[4-(trifluoro methyl)phenyl]urea;
*N*-(4-fluorophenyl)-*N*'-pyrido[1',2':1,5]pyrazolo[3,4-*d*]pyrimidin-4-ylurea;
1*H*-indole-3-carbaldehyde pyrido[1',2':1,5]pyrazolo[3,4-*d*]pyrimidin-4-ylhydrazone; and salts and solvates thereof.

The compound of formula I or salt or solvate thereof and may be employed in combination concomitantly or sequentially in any therapeutically appropriate combination. The compounds may be employed in combination in accordance with the invention by administration concomitantly in (1) a unitary pharmaceutical composition including both compounds or (2) separate pharmaceutical compositions each including one of the compounds. Alternatively, the compounds may be administered separately in a sequential manner wherein one is administered first and the other second or vice versa. Such sequential administration may be close in time or remote in time.

The cancer treatment method using the compounds of the present invention may also include administration of at least one additional cancer treatment therapy in combination concomitantly or sequentially in any therapeutically appropriate combination with the combinations of the present invention. The additional cancer treatment therapy may include radiation therapy, surgical therapy and/or at least one additional chemotherapeutic therapy including administration of at least one additional antineoplastic agent.

In a preferred embodiment, the cancer treated by the method using the compounds of the invention is breast, non-small cell lung, prostate, colorectal, renal, or bladder cancer. In another preferred embodiment, the cancer treated by the method using the compounds of the invention is mesothelioma, hepatobiliary cancer, multiple myeloma, sarcoma, or leukemia.

The kidneys are a pair of bean-shaped organs located on each side of the spine that filter blood and eliminate waste in the urine through a complex system of filtration tubules. All of the blood in the body passes through the kidneys approximately 20 times an hour. Renal cell carcinoma (RCC) is an uncommon form of cancer that is most often characterized by the presence of cancer cells in the lining of the kidney's filtration tubules. Cancer that has spread outside the kidney to several and/or distant sites in the body is referred to as metastatic RCC.

In one embodiment of the present invention, there is provided the use of a compound of formula (I) in the manufacture of a medicament for the use in the treatment of renal cell cancer comprising: administering to the mammal a compound of formula (I) or a salt or solvate thereof.

Lung cancer is the uncontrolled growth of abnormal cells in one or both of the lungs. While normal lung tissue cells reproduce and develop into healthy lung tissue, these abnormal cells reproduce rapidly and never grow into normal lung tissue. Lumps of cancer cells (tumors) then form and disrupt the lung, making it difficult to function properly. Though there are many forms of lung cancer, most are classified as one of two types: small cell lung cancer or non-small cell lung cancer (NSCLC). Of these, approximately 75% are NSCLC.

More than 87% of lung cancers are smoking related. However, not all smokers develop lung cancer. Quitting smoking reduces an individual's risk significantly, although former smokers remain at greater risk for lung cancer than people who never smoked. Exposure to other carcinogens such as asbestos and radon gas also increases an individual's risk, especially when combined with cigarette or cigar smoking. For example, most patients with mesothelioma, a rare form of cancer where malignant cells are found in the sac lining the chest (the pleura), the lining of the abdominal cavity (the peritoneum) or the lining around the heart (the pericardium), have been exposed at some time in their lives to asbestos in the workplace or at home.

In another embodiment of the present invention, there is provided the use of a compound of formula (I) in the manufacture of a medicament for the use in the treatment of lung cancer comprising: administering to the mammal a compound of formula (I) or a salt or solvate thereof.

Cancer of the colon or rectum is called colorectal cancer. The cancer occurs when tumors grow in the lining of the large intestine, or large bowel. Occurrence rates are similar between men and women, and risk for the cancer increases after the age of 50. Currently colorectal cancer is the fourth most common cancer in the United States.

In another embodiment of the present invention, there is provided the use of a compound of formula (I) in the manufacture of a medicament for the use in the treatment of colorectal cancer comprising: administering to the mammal a compound of formula (I) or a sals or solvate thereof.

Breast cancer is a type of cancer where cells in the breast tissue divide and grow without the normal control. About 80 percent of breast cancers originate in the mammary ducts, while about 20 percent arise in the lobules. Cancerous tumors in the breast usually grow very slowly so that by the time one is large enough to be felt as a lump, it may have been growing for as long as ten years. Though breast cancer most often occurs in women, men make up approximately one percent of those diagnosed with breast cancer.

In another embodiment of the present invention, there is provided the use of a compound of formula (I) in the manufacture of a medicament for the use in the treatment of breast cancer comprising: administering to the mammal a compound of formula (I) or a salt or solvate thereof.

Cancer of the bladder is the fourth most common malignancy among males and the tenth most common malignancy among females. Each year in the United States, over 50,000 people develop bladder cancer, of whom more than 12,000 ultimately will die of this disease.

Bladder cancer tends to occur most commonly in individuals over 60 years of age. Cigarette smoking and exposure to certain industrially used chemicals (derivatives of compounds called arylamines) are strongly associated with the development of bladder cancer. The vast majority (approximately 90%) of these cancers originate in the lining cells of the bladder, known as urothelium or transitional epithelium.

In another embodiment of the present invention, there is provided the use of a compound of formula (I) in the manufacture of a medicament for the use in the treatment of bladder cancer comprising: administering to the mammal a compound of formula (I) or a salt or solvate thereof.

Multiple myeloma (MM) is a malignant plasma cell disorder accounting for approximately 10% of hematologic malignancies (Bladé, J. et al. British Journal of Haematology, 1998: 102: 1115-23). Emerging evidence suggests that VEGF may play an important role in the pathogenesis of MM. Myeloma cells express VEGFR-1, and VEGF has been shown to induce proliferation and migration of the malignant cell (Podar, K. et al. Journal Bioligical Chemistry, 2002: 277: 7875-7881). Elevated serum levels of VEGF and basic hepatocyte growth factor (HGF) have been reported in patients with MM (Iwasaki, T et al. British Journal of Haematology, 2002: 116: 796-802). Furthermore, VEGF is expressed and secreted by myeloma cell lines and patient cells, as well as by bone marrow stromal cells (BMSCs).

In another embodiment of the present invention, there is provided the use of a compound of formula (I) in the manufacture of a medicament for the use in the treatment of multiple myeloma comprising: administering to the mammal a compound of formula (I) or a salt or solvate thereof.

Sarcoma is a general class of uncommon cancers in which the cancer cells arise from or resemble normal cells in the body known as connective tissue. Normal connective tissues include fat, muscle, blood vessels, deep skin tissues, nerves, bones, and cartilage. Cancers of cells which resemble any of these normal tissues are known as sarcomas. Sarcomas are sub-classified based upon the specific type of cell that makes up the cancer. For example, leiomyosarcoma is a malignant tumor that develops from smooth muscle tissue. Chondrosarcoma is a tumor of cells that form cartlidge.

In another embodiment of the present invention, there is provided the use of a compound of formula (I) in the manufacture of a medicament for the use in the treatment of sarcoma comprising: administering to the mammal a compound of formula (I) or a salt or solvate thereof.

Hepatobiliary tumors are abnormal growths occurring on or in the liver, bile ducts, and biliary tract, the tubes that carry bile from the liver or gallbladder to the small intestine. Approximately 1 million new cases of liver and biliary tract cancer are diagnosed around the globe every year. Incidence rates vary by country, although several areas are considered "hotspots." These include China, Japan, and Sub-Saharan Africa. It is believed the high rates in these regions may be associated with hepatitis B and/or mycotoxin contamination of foodstuffs, stored grains, drinking water, and soil.

In another embodiment of the present invention, there is provided the use of a compound of formula (I) in the manufacture of a medicament for the use in the treatment of hepatobiliary cancer comprising: administering to the mammal a compound of formula (I) or a salt or solvate thereof.

Leukemia is a cancer that starts in the white blood cells (also called WBCs or leukocytes). Blood consists of several types of cells (white blood cells, red blood cells and platelets) carried in a fluid called plasma. White blood cells help prevent and fight infection by destroying bacteria, viruses and other foreign cells. Red blood cells (called RBCs or erythrocytes) carry oxygen to all parts of the body. Platelets help control bleeding after an injury. The cells that form blood (stem cells) are produced in the bone marrow, as well as in the spleen, the lymph nodes and other organs.

In another embodiment of the present invention, there is provided the use of a compound of formula (I) in the manufacture of a medicament for the use in the treatment of leukemia comprising: administering to the mammal a compound of formula (I) or a salt or solvate thereof.

The diabetes treatment method using the compounds of the present invention may also include the administration of a compound of formula (I) or a salt or solvate thereof in combination concomitantly or sequentially in any therapeutically appropriate combination with other glucose lowering therapeutic agents. As one example, type 2 diabetes combination therapies according to the present invention would thus comprise the administration of at least one compound of formula (I) and the use of at least one other glucose lowering therapy. As a further example, combination therapies according to the present invention include the administration of at least one compound of formula (I) and at least one other glucose lowering treatment agent, for example, a sulfonourea.

While it is possible that, for use in the treatment of cancer or diabetes, a compound of formula (I) as well as salts or solvates thereof, may be administered as the raw chemical, it is possible to present the active ingredient as a pharmaceutical composition. Accordingly, the invention further provides pharmaceutical compositions, which may be administered in the methods of treating metabolic diseases or disorders of the present invention. The pharmaceutical compositions include a compound of formula (I) or salt or solvate thereof, and one or more pharmaceutically acceptable carriers, diluents, or excipients. The carrier(s), diluent(s) or excipient(s) must be acceptable in the sense of being compatible with the other ingredients of the formulation and not deleterious to the recipient thereof.

Pharmaceutical formulations may be presented in unit dose forms containing a predetermined amount of active ingredient per unit dose. Such a unit may contain, for example, 0.5mg to 1 g, preferably 1 mg to 700mg, more preferably 5mg to 100mg of a compound of formula (I), depending on the condition being treated, the route of administration and the age, weight and condition of the patient, or pharmaceutical formulations may be presented in unit dose forms containing a predetermined amount of active ingredient per unit dose. Preferred unit dosage formulations are those containing a daily dose or sub-dose, as herein above recited, or an appropriate fraction thereof, of an active ingredient. Furthermore, such pharmaceutical formulations may be prepared by any of the methods well known in the pharmacy art.

The compound of formula (I) may be administered by any appropriate route. Suitable routes include oral, rectal, nasal, topical (including buccal and sublingual), vaginal, and parenteral (including subcutaneous, intramuscular, intraveneous, intradermal, intrathecal, and epidural). It will be appreciated that the preferred route may vary with, for example, the condition of the recipient.

Pharmaceutical formulations adapted for oral administration may be presented as discrete units such as capsules or tablets; powders or granules; solutions or suspensions in aqueous or non-aqueous liquids; edible foams or whips; or oil-in-water liquid emulsions or water-in-oil liquid emulsions.

For instance, for oral administration in the form of a tablet or capsule, the active drug component can be combined with an oral, non-toxic pharmaceutically acceptable inert carrier such as ethanol, glycerol, water and the like. Powders can be prepared by comminuting the compound to a suitable fine size and mixing with a similarly comminuted pharmaceutical carrier such as an edible carbohydrate, as, for example, starch or mannitol. Flavoring, preservative, dispersing and coloring agent can also be present.

Capsules can be made by preparing a powder mixture as described above, and filling formed gelatin sheaths. Glidants and lubricants such as colloidal silica, talc, magnesium stearate, calcium stearate or solid polyethylene glycol can be added to the powder mixture before the filling operation. A disintegrating or solubilizing agent such as agar-agar, calcium carbonate or sodium carbonate can also be added to improve the availability of the medicament when the capsule is ingested.

Moreover, when desired or necessary, suitable binders, lubricants, disintegrating agents and coloring agents can also be incorporated into the mixture. Suitable binders include starch, gelatin, natural sugars such as glucose or betalactose, corn sweeteners, natural and synthetic gums such as acacia, tragacanth or sodium alginate, carboxymethylcellulose, polyethylene glycol, waxes and the like. Lubricants used in these dosage forms include sodium oleate, sodium stearate, magnesium stearate, sodium benzoate, sodium acetate, sodium chloride and the like. Disintegrators include, without limitation, starch, methyl cellulose, agar, bentonite, xanthan gum and the like. Tablets can be formulated, for example, by preparing a powder mixture, granulating or slugging, adding a lubricant and disintegrant and pressing into tablets. A powder mixture is prepared by mixing the compound, suitably comminuted, with a diluent or base as described above, and optionally, with a binder such as carboxymethylcellulose, an aliginate, gelatin, or polyvinyl pyrrolidone, a solution retardant such as paraffin, a resorption accelerator such as a quaternary salt and/or an absorption agent such as bentonite, kaolin or dicalcium phosphate. The powder mixture can be granulated by wetting with a binder such as syrup, starch paste, acadia mucilage or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc or mineral oil. The lubricated mixture is then compressed into tablets. The compounds of the present invention can also be combined with free flowing inert carrier and compressed into tablets directly without going through the granulating or slugging steps. A clear or opaque protective coating consisting of a sealing coat of shellac, a coating of sugar or polymeric material and a polish coating of wax can be provided. Dyestuffs can be added to these coatings to distinguish different unit dosages.

Oral fluids such as solution, syrups and elixirs can be prepared in dosage unit form so that a given quantity contains a predetermined amount of the compound. Syrups can be prepared by dissolving the compound in a suitably flavored aqueous solution, while elixirs are prepared through the use of a non-toxic alcoholic vehicle. Suspensions can be formulated by dispersing the compound in a non-toxic vehicle. Solubilizers and emulsifiers such as ethoxylated isostearyl alcohols and polyoxy ethylene sorbitol ethers, preservatives, flavor additive such as peppermint oil or natural sweeteners or saccharin or other artificial sweeteners, and the like can also be added.

Where appropriate, dosage unit formulations for oral administration can be microencapsulated. The formulation can also be prepared to prolong or sustain the release as for example by coating or embedding particulate material in polymers, wax or the like.

The agents for use according to the present invention can also be administered in the form of liposome delivery systems, such as small unilamellar vesicles, large unilamellar vesicles and multilamellar vesicles. Liposomes can be formed from a variety of phospholipids, such as cholesterol, stearylamine or phosphatidylcholines.

Agents for use according to the present invention may also be delivered by the use of monoclonal antibodies as individual carriers to which the compound molecules are coupled. The compounds may also be coupled with soluble polymers as targetable drug carriers. Such polymers can include polyvinylpyrrolidone, pyran copolymer, polyhydroxypropylmethacrylamide-phenol, polyhydroxyethylaspartamidephenol, or polyethyleneoxidepolylysine substituted with palmitoyl residues. Furthermore, the compounds may be coupled to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates and cross-linked or amphipathic block copolymers of hydrogels.

Pharmaceutical formulations adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6), 318 (1986).

Pharmaceutical formulations adapted for topical administration may be formulated as ointments, creams, suspensions, lotions, powders, solutions, pastes, gels, sprays, aerosols or oils.

For treatments of the eye or other external tissues, for example mouth and skin, the formulations are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base.

Pharmaceutical formulations adapted for topical administrations to the eye include eye drops wherein the active ingredient is dissolved or suspended in a suitable carrier, especially an aqueous solvent.

Pharmaceutical formulations adapted for topical administration in the mouth include lozenges, pastilles and mouth washes.

Pharmaceutical formulations adapted for rectal administration may be presented as suppositories or as enemas.

Pharmaceutical formulations adapted for nasal administration wherein the carrier is a solid include a coarse powder having a particle size for example in the range 20 to 500 microns which is administered in the manner in which snuff is taken, i.e. by rapid inhalation through the nasal passage from a container of the powder held close up to the nose. Suitable formulations wherein the carrier is a liquid, for administration as a nasal spray or as nasal drops, include aqueous or oil solutions of the active ingredient.

Pharmaceutical formulations adapted for administration by inhalation include fine particle dusts or mists that may be generated by means of various types of metered dose pressurised aerosols, nebulizers or insufflators.

Pharmaceutical formulations adapted for vaginal administration may be presented as pessaries, tampons, creams, gels, pastes, foams or spray formulations.

Pharmaceutical formulations adapted for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets.

It should be understood that in addition to the ingredients particularly mentioned above, the formulations may include other agents conventional in the art having regard to the type of formulation in question, for example those suitable for oral administration may include flavoring agents.

The present invention provides the use of a compound of formula (I) for the treatment of several conditions or diseases, all of which comprise the step of administering a compound of formula (I). As used herein, the term "treatment" refers to alleviating the specified condition, eliminating or reducing the symptoms of the condition, slowing or eliminating the progression of the condition and preventing or delaying the initial occurrence of the condition in a subject, or reoccurrance of the condition in a previously afflicted subject.

It will be appreciated by those skilled in the art that the amount of a compound of formula (I) required for use in treatment will vary with the nature of the condition being treated and the age and the condition of the patient and will be ultimately at the discretion of the attendant physician or veterinarian. Typically, the compound of formula (I) will be given in the range of 0.1 to 100 mg/kg body weight of recipient (mammal) per day and more usually in the range of 1 to 10 mg/kg body weight per day. The desired dose may conveniently be presented in a single dose or as divided doses administered at appropriate intervals, for example as two, three, four or more sub-doses per day.

The compounds of this invention may be made by a variety of methods. Illustrative general synthetic methods are set out below and then specific compounds of the invention are prepared in the working Examples.

In all of the examples described below, protecting groups for sensitive or reactive groups are employed where necessary in accordance with general principles of synthetic chemistry. Protecting groups are manipulated according to standard methods of organic synthesis (T. W. Green and P. G. M. Wuts (1991) Protecting Groups in Organic Synthesis, John Wiley & Sons, incorporated by reference with regard to protecting groups). These groups are removed at a convenient stage of the compound synthesis using methods that are readily apparent to those skilled in the art. The selection of processes as well as the reaction conditions and order of their execution shall be consistent with the preparation of compounds of formula (I).

Those skilled in the art will recognize if a stereocenter exists in compounds of formula (I). Accordingly, the present invention includes all possible stereoisomers and includes not only racemic compounds but the individual enantiomers as well. When a compound is desired as a single enantiomer, such may be obtained by stereospecific synthesis, by resolution of the final product or any convenient intermediate, or by chiral chromatographic methods as are known in the art. Resolution of the final product, an intermediate, or a starting material may be effected by any suitable method known in the art. See, for example, Stereochemistry of Organic Compounds by E. L. Eliel, S. H. Wilen, and L. N. Mander (Wiley-Interscience, 1994).

### ABBREVIATIONS

As used herein the symbols and conventions used in these processes, schemes and examples are consistent with those used in the contemporary scientific literature, for example, the *Journal of the American Chemical Society* or the *Journal of Biological Chemistry.* Unless otherwise noted, all starting materials were obtained from commercial suppliers and used without further purification. Specifically, the following abbreviations may be used in the examples and throughout the specification:

| | |
|---|---|
| g (grams); | mg (milligrams); |
| L (liters); | mL (milliliters); |
| µL (microliters); | psi (pounds per square inch); |
| M (molar); | mM (millimolar); |
| N (Normal); | Kg (kilogram); |
| Hz (Hertz); | MHz (megahertz); |
| mol (moles); | mmol (millimoles); |
| RT (room temperature); | min (minutes); |
| h (hours); | mp (melting point); |
| Tᵣ (retention time); | RP (reverse phase); |
| DCM (dichloromethane); | DCE (dichloroethane); DMF |
| (*N,N*-dimethylformamide); HOAc (acetic acid); | |
| TMSE (2-(trimethylsilyl)ethyl); | TMS (trimethylsilyl); |
| TIPS (triisopropylsilyl); | TBS (*t*-butyldimethylsilyl); |
| THF (tetrahydrofuran); | DMSO (dimethylsulfoxide); |
| EtOAc (ethyl acetate); | DME (1,2-dimethoxyethane); |
| TFA (trifluoroacetic acid); | TLC (thin layer chromatography); |
| HPLC (high pressure liquid chromatography); | |
| EDTA (ethylenediaminetetraacetic acid); | |

Unless otherwise indicated, all temperatures are expressed in °C (degrees Centigrade). All reactions conducted under an inert atmosphere at room temperature unless otherwise noted.

The 1 H NMR spectra were recorded on a Varian VXR-300, a Varian Unity-300, a Varian Unity-400, a Varian Unity-INOVA-300, a Varian Unity-INOVA-400, or a Mercury VX-400 instrument. Chemical shifts are expressed in parts per million (ppm, δ units). Splitting patterns are designated as s, singlet; d, doublet; t, triplet; q, quartet; m, multiplet; br, broad; hept, heptuplet.

Low-resolution mass spectra (MS) were recorded on a JOEL JMS-AX505HA, a JOEL SX-102, a SCIEX-APliii, a Waters ZQ, a Waters ZMO, a Waters Quattro Micro, or Waters GCT spectrometers. All mass spectra were taken under electrospray ionization (ES, either in the positive ion mode or negative ion mode), atmospheric pressure chemical ionization (APCI), or by fast atom bombardment (FAB) methods. Infrared (IR) spectra were obtained on a Nicolet 510 FT-IR spectrometer using a 1-mm NaCl cell. All reactions were monitored by thin-layer chromatography on 0.25 mm E. Merck silica gel plates (60F-254), visualized with UV light, iodine staining, or 7% ethanolic phosphomolybdic acid or p-anisldehyde solutions. Flash column chromatography was performed on silica gel (230-400 mesh, Merck).

Analytical purity was assessed on a Hewlett Packard series 1050 or 1100 system equipped with a diode array spectrometer. The stationary phase was either a Dynamax C8 column (25 cm x 4.1 mm), a Dynamax 60A C18 column (25 cm x 4.6 mm), a Vydac C18 column (5m, 4.6 mm X 250 mm), a Supelco C18 column (5m, 4.6 mm X 150 mm), or a Rainin C18 column (5m, 4.6 mm X 250 mm). The flow rate was 1.0 to 1.5 mL/min. (t0 = 2.8 or 3.0 min.) and the solvent systems were as described below. Enantiomeric purity was assessed using either a Chiralpak AD column (25 cm x 4.6 mm) or a Chiralpak OD column (25cm x 4.6 mm) on either a Hewlet Packard series 1050 HPLC system equipped with a diode array spectrometer or on a Supercritical Fluid (SFC) system using CO₂ / methanol as the mobile phase.

### Method of Synthesis

The compounds of formula (I) can be prepared readily according to the following reaction schemes (in which all variables are as defined before) and Examples or modifications thereof using readily available starting materials, reagents, and conventional systhesis procedures. In these reactions, it is also possible to make use of variants which are themselves known to those of ordinary skill in this art, but are not mentioned in greater detail.

The aminonitrile intermediate 1 (2-amino-3-cyano-pyrazolo[1,5-a]pyridine) was synthesized from the commercially available N-aminopyridinium iodide and malononitrile (Scheme 1). Combining these reagents in ethanol and heating with microwave irradiation in the presence of two equivalents of potassium carbonate generates, after silica gel chromatography, the desired product. 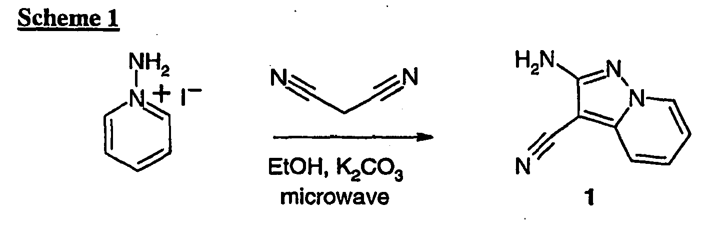 This intermediate can be used to prepare anilino-substituted derivatives and urea-substituted derivatives as shown in Schemes 2 and 3, respectively. Thus, heating the aminonitrile (1) in formic acid containing a catalytic amount of concentrated sulfuric acid provided the pyrimidinone (2). Conversion to the chloride (3) can be realized by heating in neat phosphorus oxychloride. The final tricyclic aniline (4) can be prepared by displacement of the chloride with an aniline. This can be accomplished by combining the reagents in an appropriate solvent and heating with microwave irradiation (Scheme 2). 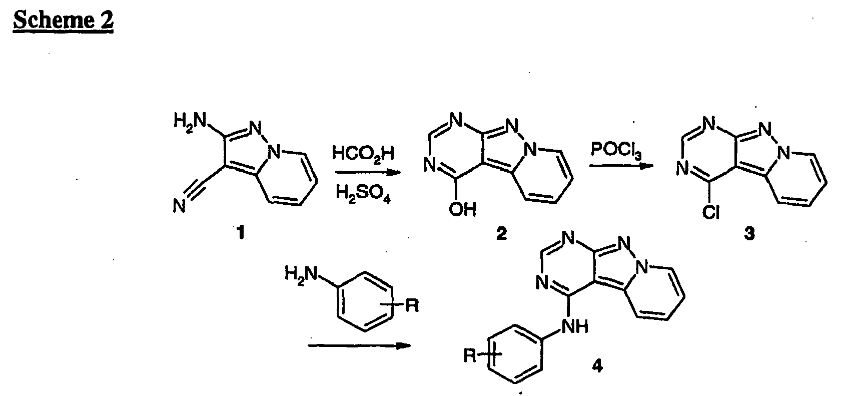 Preparation of the urea substituted derivatives can be accomplished using microwave irradiation by heating the aminonitrile (1) in formamide. The resulting amine (5) can be condensed with an isocyanate resulting in a tricyclic urea (6) as depicted in Scheme 3.

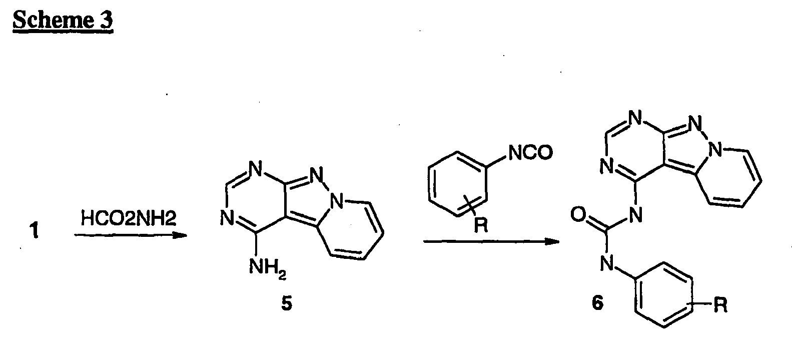

The following examples are intended for illustration only and are not intended to limit the scope of the invention in any way.

### EXAMPLES

### Example 1: pyrido[1',2':1,5]pyrazolo[3,4-d]pyrimidin-4-amine

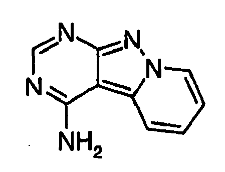

### Step 1. 2-aminopyrazolo[1,5-a]pyridine-3-carbonitrile

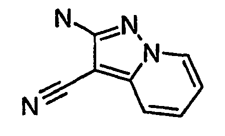

A round-bottomed flask was charged with N-aminopyridinium iodide (2.20g), potassium carbonate (2.76g), malononitrile (0.66g) and ethanol (100mL). The mixture was heated to reflux for several hours. The ethanol was removed under vacuum and replaced with water. The aqueous mixture was extracted with ethyl acetate and the organics were separated and dried over magnesium sulfate. The drying salts were removed by filtration and the filtrate was concentrated to an oil. The residue was purified by silica gel chromatography to yield 2-aminopyrazolo[1,5-a]pyridine-3-carbonitrile ( 0.400g) as a tan powder. H1 NMR (d6-dmso) 8.48 (d, 1 H), 7.35-7.45 (m, 2H), 6.88 (dd, 1 H), 6.3 (br s, 2H).

### Step 2. pyrido[1',2':1,5]pyrazolo[3,4-d]pyrimidin-4-amine

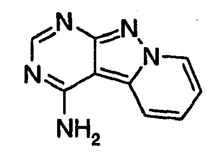

A 5mL microwave tube was charged with 2-aminopyrazolo[1,5-a]pyridine-3-carbonitrile (0.50g, from Example 1, Step 1) and formamide (3mL). The reaction was heated at 240°C for 10 minutes using microwave irradiation. The mixture was added to rapidly stirring ice water and allowed to warm to r.t. The resulting solids were collected on filter to provide pyrido[1',2':1,5]pyrazolo[3,4-*d*]pyrimidin-4-amine as a tan powder (464mg). H1 NMR (d6-dmso) 8.98 (d, 1 H), 8.61 (d, 1H), 8.31 (s, 1 H), 7.66 (dd, 1 H), 7.64 (br s, 2H), 7.37 (dd, 1 H).

### Example 2: pyrido[1',2':1,5]pyrazolo[3,4-d]pyrimidin-4(1H)-one

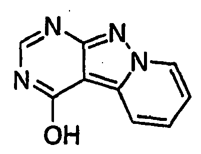

A round-bottomed flask was charged with 2-aminopyrazolo[1,5-*a*]pyridine-3-carbonitrile (1.0g, from Example 1, Step 1) and formic acid (10mL). A couple of drops of sulfuric acid (conc.) was added and the mixture was heated to 90°C for about 5h, or until consumption of starting material was complete. The reaction was cooled and diluted with diethyl ether. The resulting solids were collected on filter, washed with diethyl ether and air-dried to provide pyrido[1',2':1,5]pyrazolo[3,4-*d*]pyrimidin-4(1*H*)-one as an off-white powder (1.06g). H1 NMR (d6-dmso) 8.99 (d, 1H), 8.15 (s, 1 H), 8.09 (d, 1 H), 7.70 (dd, 1 H), 7.35 (dd, 1 H).

### Example 3: 4-chloropyrido[1',2':1,5]pyrazolo[3,4-d]pyrimidine

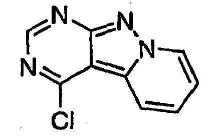

A round-bottomed flask was charged with pyrido[1',2':1,5]pyrazolo[3,4-*d*]pyrimidin-4(1 *H*)-one (1.1 g, from Example 2) and phosphorus oxychloride (50mL). The mixture was heated to 150°C until completion of reaction was evident by consumption of starting material. The mixture was cooled and diluted with diethyl ether. The resulting solids were collected on filter, washed with diethyl ether and dried under vacuum to provide 4-chloropyrido[1',2':1,5]pyrazolo[3,4-*d*]pyrimidine as an off-white powder (310mg). H1 NMR (d6-dmso) 9.31 (d, 1 H), 8.92 (s, 1H), 8.55 (d, 1 H), 8.00 (dd, 1 H), 7.75 (dd, 1H).

### Example 4: N-(3-chloro-4-fluorophenyl)pyrido[1',2':1,5]pyrazolo[3,4-d]pyrimidin-4-amine

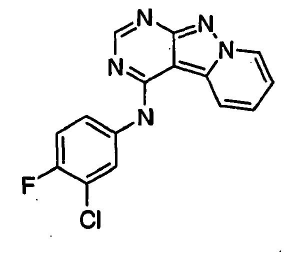

A round-bottomed flask was charged with 4-chloropyrido[1',2':1,5]pyrazolo[3,4-*d*]pyrimidine (40mg, from Example 3), isopropanol (10mL) and 3-chloro-4-fluoroaniline (28.5mg). To this mixture was added a drop of hydrochloric acid (conc.). The reaction was heated to reflux until consumption of starting material was complete. The isopropanol was removed under vacuum and replaced with a saturated solution of sodium bicarbonate. The aqueous mixture was extracted with ethyl acetate and the organic extracts were dried over magnesium sulfate. The drying salts were removed by filtration and the filtrate was concentrated to dryness. The residue was triturated with diethyl ether and the resulting solids were collected on filter to provide *N*-(3-chloro-4-fluorophenyl)-pyrido[1',2':1,5]pyrazolo[3,4-*d*]pyrimidin-4-amine (15mg) as a powder. H1 NMR (d6-dmso) 9.20 (br s, 1 H), 9.05 (d, 1 H), 8.74 (d, 1 H), 8.54 (s, 1 H), 8.06 (d, 1 H), 7.74-7.84 (m, 2H), 7.47 (dd, 1 H), 7.40 (m, 1 H). Mass (ES- 312, 314).

### Example 5: N-(3-chloro-4-{[(3-fluorophenyl)methyl]oxy}phenyl)-pyrido[1',2':1,5] pyrazolo[3,4-d]pyrimidin-4-amine

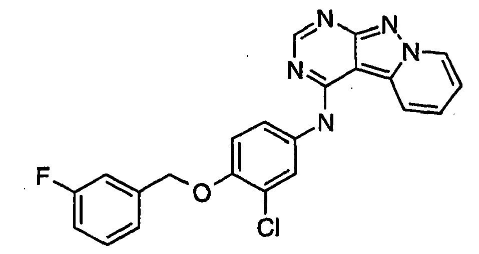

The same procedure as that for Example 4 was used, except 3-chloro-4-fluoroaniline was replaced with 3-chloro-4-{[(3-fluorophenyl)methyl]oxy}aniline to provide *N*-(3-chloro-4-{[(3-fluorophenyl)methyl]oxy}phenyl)-pyrido[1',2':1,5]pyrazolo[3,4-*d*]pyrimidin-4-amine. 1 H NMR (300 MHz, DMSO-*d*₆) d ppm 9.21 (s, 1 H) 9.12 (d, *J*=6.59 Hz, 1 H) 8.83 (d, *J*=8.79 Hz, 1 H) 8.53 (s, 1 H) 7.92 (m, 1 H) 7.81 (m, *J*=6.39, 6.39 Hz, 1 H) 7.68 (m, 1 H) 7.40 (m, 6 H) 5.27 (s, 2 H) Mass (ES+ 402, 404).

### Example 6: N-{3-chloro-4-[(phenylmethyl)oxy]phenyl}pyrido[1',2':1,5]pyrazolo[3,4-d]pyrimidin-4-amine

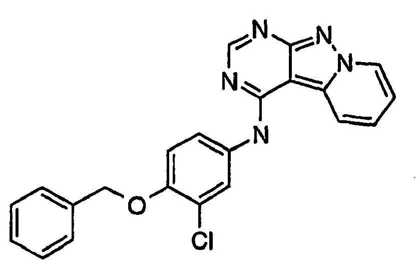

The same procedure as that for Example 4 was used, except 3-chloro-4-fluoroaniline was replaced with {3-chloro-4-[(phenylmethyl)oxy]phenyl}amine to provide *N*-{3-chloro-4-[(phenylmethyl)oxy]phenyl}pyrido[1',2':1,5]pyrazolo[3,4-*d*]pyrimidin-4-amine. 1H NMR (400 MHz, Acetone) δ ppm 8.94 (d, *J*=6.77 Hz, 1 H) 8.58 (d, *J*=8.61 Hz, 1 H) 8.54 (s, 1 H) 8.41 (br. s., 1 H) 8.00 (d, *J*=2.56 Hz, 1 H) 7.98 (s, 1 H) 7.71 (m, 2 H) 7.52 (m, 2 H) 7.44 (m, 1 H) 7.39 (m, 1 H) 7.32 (m, 1 H) 7.20 (d, *J*=8.79 Hz, 1 H) 5.23 (s, 2 H) Mass (ES+ 401, 403).

### Example 7: N-[2-(phenylmethyl)-1H-benzimidazol-5-yl]pyrido[1',2':1,5]pyrazolo-[3,4-d]pyrimidin-4-amine

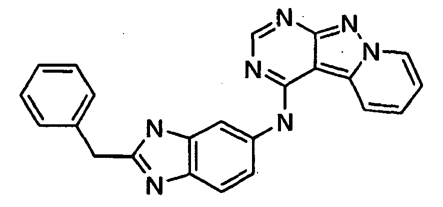

The same procedure as that for Example 4 was used, except 3-chloro-4-fluoroaniline was replaced with 2-(phenylmethyl)-1*H*-benzimidazol-5-amine to provide *N*-[2-(phenylmethyl)-1*H*-benzimidazol-5-yl]pyrido[1',2':1,5]pyrazolo-[3,4-*d*]pyrimidin-4-amine. 1 H NMR (300 MHz, DMSO-*d*₆) δ ppm 9.16 (d, *J*=6.32 Hz, 1 H) 8.85 (s, 1 H) 8.56 (s, 1 H) 8.13 (m, 1 H) 7.86 (m, 1 H) 7.70 (m, 2 H) 7.55 (m, 1 H) 7.37 (m, 6 H) 4.41 (s, 2 H) Mass (ES+ 392).

### Example 8: N-(3-cyanophenyl)-N'-pyrido[1',2':1,5]pyrazolo[3,4-d]pyrimidin-4-ylurea

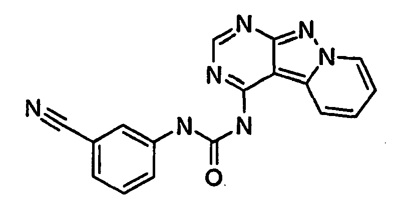

Pyrido[1',2':1,5]pyrazolo[3,4-*d*]pyrimidin-4-amine (0.06g, 0.32mmol) was stirred with 3-isocyanatobenzonitrile (0.093g) in 5mL of acetonitrile at 60°C for 16 hrs. The mixture was then concentrated to dryness and the residue was triturated from hot methanol to provide *N*-(3-cyanophenyl)-*N*'-pyrido[1',2':1,5]pyrazolo[3,4-*d*]pyrimidin-4-ylurea as a white solid (0.022g). 1 H NMR (400 MHz, DMSO-*d*₆) δ ppm 9.09 (d, *J*=6.77 Hz, 1 H) 8.55 (m, 1 H) 8.33 (m, 1 H) 8.25 (s, 1 H) 7.90 (m, *J*=8.61 Hz, 1 H) 7.84 (dd, *J*=7.69 Hz, 1 H) 7.53 (dd, *J*=7.87 Hz, 1 H) 7.45 (m, 2 H)

### Example 9: N-pyrido[1',2':1,5]pyrazolo[3,4-d]pyrimidin-4-yl-N'-[3-(trifluoromethyl)phenyl]urea

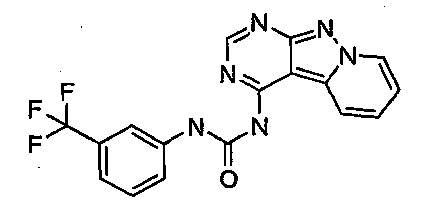

Pyrido[1',2':1,5]pyrazolo[3,4-*d*]pyrimidin-4-amine (0.06g, 0.32mmol) was stirred with 1-isocyanato-3-(trifluoromethyl)benzene (0.049mL) in 5mL of acetonitrile at 60°C for 16 hrs. The mixture was then concentrated to dryness and the residue was triturated from hot methanol to provide *N*-pyrido[1',2':1,5]pyrazolo[3,4-*d*]pyrimidin-4-yl-*N*'-[3-(trifluoromethyl)phenyl]urea as a white solid (0.036g, 0.10mmol). 1 H NMR (300 MHz, DMSO-*d*₆) δ ppm 13.3 (s, 1 H) 9.96 (s, 1 H) 9.08 (d, *J*=7.01 Hz, 1 H) 8.54 (m, *J*=7.83 Hz, 1 H) 8.31 (m, *J*=15.25 Hz, 2 H) 7.81 (m, 2 H) 7.53 (dd, *J*=8.24 Hz, 1 H) 7.44 (m, 1 H) 7.32 (d, *J*=8.11 Hz, 1 H)

### Example 10: N-(3-nitrophenyl)-N'-pyrido[1',2':1,5]pyrazolo[3,4-d]pyrimidin-4-ylurea

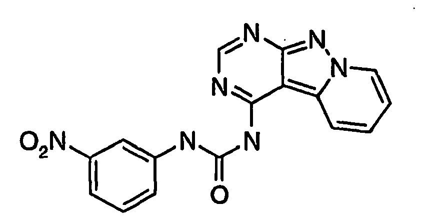

Pyrido[1',2':1,5]pyrazolo[3,4-*d*]pyrimidin-4-amine (0.06g, 0.32mmol) was stirred with 1-isocyanato-3-nitrobenzene (0.053mg) in 5mL of acetonitrile at 60°C for 16 hrs. The mixture was then concentrated to dryness and the residue was triturated from hot methanol to provide *N*-(3-nitrophenyl)-*N*'-pyrido[1',2':1,5]pyrazolo[3,4-*d*]pyrimidin-4-ylurea as a white solid (0.01 mmol, 0.036g). 1 H NMR (400 MHz, DMSO-*d*₆) δ ppm 9.40 (s, 1 H) 9.01 (d, *J*=6.77 Hz, 1 H) 8.64 (d, *J*=8.42 Hz, 1 H) 8.55 (m, 1 H) 8.34 (s, 1 H) 7.85 (dd, *J*=7.96, 1.74 Hz, 2 H) 7.77 (m, 1 H) 7.70 (m, 1 H) 7.58 (m, *J*=8.15, 8.15 Hz, 1 H) 7.39 (m, 1 H)

### Example 11: N-(4-bromophenyl)-N'-pyrido[1',2':1,5]pyrazolo[3,4-d]pyrimidin-4-ylurea

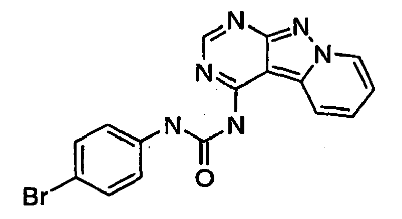

Pyrido[1',2':1,5]pyrazolo[3,4-*d*]pyrimidin-4-amine (0.06g, 0.32mmol) was stirred with 1-bromo-4-isocyanatobenzene (0.064mg) in 5mL of acetonitrile at 60°C for 16 hrs. The mixture was then concentrated to dryness and the residue was triturated from hot methanol to provide *N*-(4-bromophenyl)-*N*'-pyrido[1',2':1,5]pyrazolo[3,4-*d*]pyrimidin-4-ylurea as a white solid (0.03mmol, 0.011g). 1H NMR (300 MHz, DMSO-*d*₆) δ ppm 9.07 (d, *J*=7.01 Hz, 1 H) 8.55 (m, *J*=9.75 Hz, 1 H) 8.35 (s, 1 H) 7.81 (m, 1 H) 7.67 (d, *J*=8.93 Hz, 2 H) 7.45 (m, 3 H) Mass (ES- 380, 382).

### Example 12: N-pyrido[1',2':1,5]pyrazolo[3,4-d]pyrimidin-4-yl-N'-[4-(trifluoro methyl)phenyl]urea

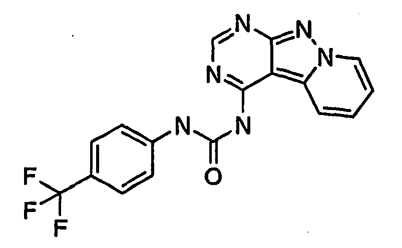

Pyrido[1',2':1,5]pyrazolo[3,4-*d*]pyrimidin-4-amine (0.06g, 0.32mmol) was stirred with 1-isocyanato-4-(trifluoromethyl)benzene (0.049mL) in 5mL of acetonitrile at 60°C for 16 hrs. The mixture was then concentrated to dryness and the residue was triturated from hot methanol to provide *N*-pyrido[1',2':1,5]pyrazolo[3,4-*d*]pyrimidin-4-yl-*N*'-[4-(trifluoro methyl)phenyl]urea as a white solid (0.016g 0.04mmol). 1 H NMR (300 MHz, DMSO-*d*₆) δ ppm 13.30 (m, 1 H) 9.95 (m, 1 H) 9.08 (d, *J*=7.01 Hz, 1 H) 8.54 (d, *J*=7.83 Hz, 1 H) 8.32 (m, 2 H) 7.82 (m, 2 H) 7.52 (m, 1 H) 7.44 (m, 1 H) 7.32 (d, *J*=8.11 Hz, 1 H)

### Example 13: N-(4-fluorophenyl)-N'-pyrido[1',2':1,5]pyrazolo[3,4-d]pyrimidin-4-ylurea

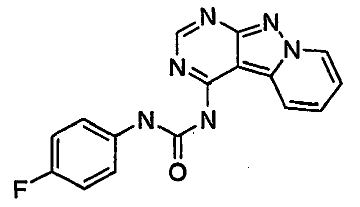

Pyrido[1',2':1,5]pyrazolo[3,4-*d*]pyrimidin-4-amine (0.025g, 0.14mmol) was stirred with 1-isocyanato-4-(trifluoromethyl)benzene (0.017uL) in 5mL of acetonitrile at 60°C for 16 hrs. The mixture was then concentrated to dryness and the residue was triturated from hot methanol to provide *N*-(4-fluorophenyl)-*N*'-pyrido[1',2':1,5]pyrazolo[3,4-*d*]pyrimidin-4-ylurea as a white solid (0.009g). 1 H NMR (300 MHz, DMSO-*d*₆) δ ppm 7.18 (m, 2 H) 7.46 (m, 1 H) 7.74 (m, 2 H) 7.85 (m, 1 H) 8.33 (m, 1 H) 8.55 (m, 1 H) 9.11 (m, 1 H) 9.72 (m, 1 H) 13.31 (m, 1 H). MS (M+1) 323.

### Example 14: 1H-indole-3-carbaldehyde pyrido[1',2':1,5]pyrazolo[3,4-d]pyrimidin-4-ylhydrazone

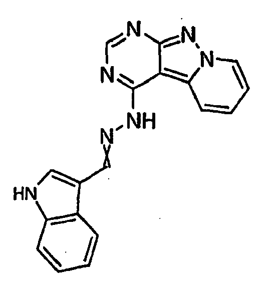

A solution of 4-chloropyrido[1',2':1,5]pyrazolo[3,4-d]pyrimidine (0.074g, 0.36mmol) in isopropylalcohol (5mL) is treated with hydrazine monohydrate (0.035mL, 0.73mmol) before heating to reflux. After 1 hour, solids are collected and rinsed with diethylether. These solids are then treated with isopyopylalcohol (5mL) and 1 H-indole-3-carbaidehyde (0.039mg, 0.26mmol) and heated to 60°C for 3 hours. The mixture was concentrated and triturated with diethylether to afford product as a tan solid (0.002mg, 0.006mmol). 1 H NMR (300 MHz, DMSO-d₆) d ppm 11.59 (br. s., 1 H) 11.17 (m, J=3.02 Hz, 1 H) 8.87 (d, J=6.87 Hz, 1 H) 8.70 (s, 1 H) 8.51 (d, J=6.05 Hz, 1 H) 8.08 (d, J=8.66 Hz, 1 H) 8.01 (d, J=3.57 Hz, 1 H) 7.86 (d, J=2.61 Hz, 1 H) 7.59 (m, 1 H) 7.44 (m, J=6.87 Hz, 1 H) 7.20 (m, J=6.25, 6.25 Hz, 2 H) MS (M+1) 328

### Biological Data

### Type 1 Receptor Tyrosine Kinase Assays-- Enzyme Assays:

Compounds of the present invention were tested for EGFR or ErbB-2 protein tyrosine kinase inhibitory activity in substrate phosphorylation assays using enzymes purified from a baculovirus expression system. Reagent production and assay methodology were conducted essentially as described (Brignola, P.S., et al, (2002) J. Biol. Chem. v. 277, 1576-1585).

The method measures the ability of the isolated enzyme to catalyse the transfer of the γ-phosphate from ATP onto tyrosine residues in a biotinylated synthetic peptide (biotin-Ahx-RAHEEIYHFFFAKKK-amide). Reactions were performed in 96 or 384 well polystyrene plates in a final volume of 20 or 45µl. Reaction mixtures contained 50mM MOPS (pH 7.5), 2mM MnCl₂, 10µM ATP, 0.125 µCi [γ-³³p] ATP per reaction, 2 µM peptide substrate, and 1 mM dithiothreitol. Reactions were initiated by adding 1 pmol (20nM) per reaction of the indicated enzyme. The reaction was allowed to proceed for 15 minutes, terminated and quantified using a scintillation proximity assay procedure as described (McDonald, O.B., Antonsson, B., Arkinstal, S., Marshall, C.J., and Wood, E.R. (1999) Analytical Biochemistry, 268, 318-329).

The catalytic domains of vascular endothelial growth factor receptor 2 (VEGFR2) was expressed and purified using methods similar to those described for ErbB-2 and EGFR. Kinase assays were performed as described above with the following modifications: VEGFR2 assays contained 10 nM enzyme, 100 mM HEPES, pH 7.5, 0.1 mg/ml bovine serum albumin, 0.1 mM dithiothreitol, 360 nM peptide A, 75 µM ATP, and 5 mM MgCl₂. The reaction was allowed to proceed for 40 min. Product was detected using a homogeneous time-resolved fluorescence procedure (Park, Y.-W., Cummings, R. T., Wu, L., Zheng, S., Cameron, P. M., Woods, A., Zaller, D. M., Marcy, A. I., and Hermes, J. D. (1999) Anal. Biochem. 269, 94-104). Briefly, the reactions were quenched by adding 100 µl of 100 mM HEPES, pH 7.5, 100 mM EDTA, 45 nM streptavidin-linked allophycocyanin (Molecular Probes, Eugene, OR), and 3 nM europium-conjugated anti-phosphotyrosine antibody (Wallac, Turku, Finland). The product was detected using a Victor plate reader (Wallac, Turku, Finland) with a time delay at 665 nm.

Human GSK3b was expressed in *E. coli* with a 6-His tag at the N-terminus. The protein was purified using metal-chelate affinity chromatography. The incorporation of radioactive phosphate into a biotinylated synthetic peptide, Biotin-Ahx-AAAKRREILSRRPS(PO3)YR-amide was detected using a scintillation proximity assay (SPA) method as described above. Assay conditions were as follows: 100 mM HEPES, pH 7.2, 10 mM MgCl2, 0.3 mg/mL heparin sulfate, 0.1 mg/mL BSA , 1 mM DTT , 2.5 uM ATP, 0.6 uCi/rxn ³³P-ATP, 1.2 ug/mL GSK-3b protein. The plates are incubated at room temperature for 19 minutes prior to the addition of SPA stop solution.

**Data Calculations -** Peptide substrate phosphorylation reactions were performed as described above. The example compound indicated was serially diluted 1 to 3 and added to the reactions to produce an 11-point dose-response curve ranging from 0.0001 to 10µM. The IC₅₀ values were estimated from data fit to the equation *y* = Vₘₐₓ·(1 - (*x*/(K + *x*)). "+/-" represents IC₅₀ values >10µM, "+" represents IC₅₀ values 1-10µM, "++" represents IC₅₀ values 0.1-1µM, and "+++" represents IC₅₀ values <0.1µM in the above described assays. Results are indicated in table 1.

**Table 1**

| Example | ErbB2 | EGFR | GSK3 | VEGF-R |
|---|---|---|---|---|
| 1 | +/- | +/- | +/- | +/- |
| 4 | +/- | ++ | +/- | +/- |
| 5 | +++ | +++ | +/- | +/- |
| 6 | ++ | +++ | +/- | +/- |
| 7 | + | + | +/- | +/- |
| 8 | +/- | +/- | ++ | ++ |
| 9 | +/- | +/- | ++ | ++ |
| 10 | +/- | +/- | ++ | +/- |
| 11 | +/- | +/- | + | + |
| 12 | +/- | +/- | + | + |
| 13 | +/- | +/- | +/- | ++ |
| 14 | +/- | + | + | +/- |

## Claims

1. A compound of formula (I) 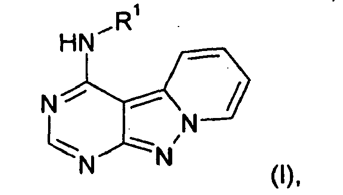 or salts and solvates thereof, wherein
R¹ is H, substituted aryl, substituted heteroaryl, -C(O)N(H)R², or - N=CR³, wherein when R¹ is substituted aryl or substituted heteroaryl, each substituent is independently selected from the group consisting of aryl, halo, and -OR⁴;
R² is substituted aryl, wherein each substituent is independently selected from the group consisting of cyano, halo, nitro, and haloalkyl;
R³ is heteroaryl; and
R⁴ is aryl or haloaryl.

2. A compound as claimed in claim 1, wherein R¹ is H.

3. A compound as claimed in claim 1, wherein R¹ is substituted aryl, or substituted heteroaryl, wherein each substituent is independently selected from the group consisting of aryl, halo, and -OR⁴; and
R⁴ is aryl or haloaryl.

4. A compound as claimed in claim 1, wherein R¹ is -C(O)N(H)R²; and R² is substituted aryl, wherein each substituent is independently selected from the group consisting of cyano, halo, nitro, and haloalkyl.

5. A compound of claim 1, wherein said compound is selected from: 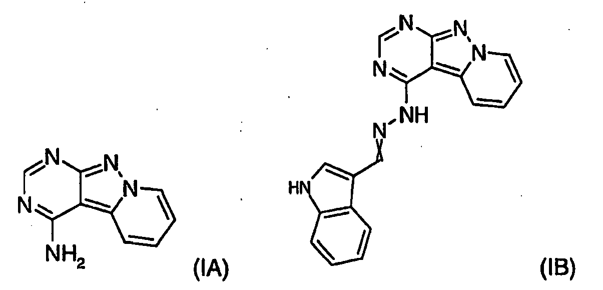 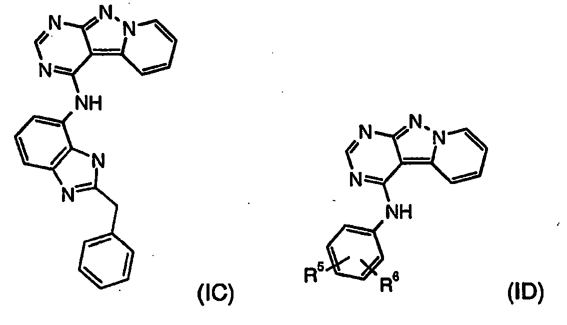 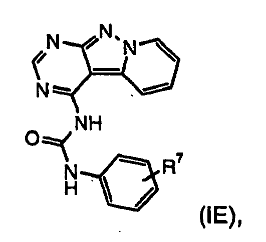 and salts and solvates thereof,
wherein R⁵ and R⁶ are independently selected from the group consisting of hydrogen, halo, and -OR⁴;
R⁴ is aryl or haloaryl; and
R⁷ is cyano, halo, nitro, and haloalkyl.

6. A compound selected from:
pyrido[1',2':1,5]pyrazolo[3.4-*d*]pyrimidin-4-amine;
*N*-(3-chloro-4-fluorophenyl)pyrido[1',2':1,5]pyrazolo[3,4-*d*]pyrimidin-4-amine;
*N*-(3-chloro-4-{[(3-fluorophenyl)methyl]oxy}phenyl)-pyrido[1',2':1,5] pyrazolo[3,4-*d*]pyrimidin-4-amine;
*N*-{3-chloro-4-[(phenylmethyl)oxy]phenyl}pyrido[1',2':1,5]pyrazolo[3,4-*d*]pyrimidin-4-amine;
*N*-[2-(phenylmethyl)-1*H*-benzimidazol-5-yl]pyrido[1',2':1,5]pyrazolo-[3,4-*d*]pyrimidin-4-amine;
*N*-(3-cyanophenyl)-*N*'-pyrido[1',2':1,5]pyrazolo[3,4-*d*]pyrimidin-4-ylurea;
*N*-pyrido[1',2':1,5]pyrazolo[3,4-*d*]pyrimidin-4-yl-*N*'-[3-(trifluoromethyl)phenyl]urea;
*N*-(3-nitrophenyl)-*N*'-pyrido[1',2':1,5]pyrazolo[3,4-*d*]pyrimidin-4-ylurea;
*N*-(4-bromophenyl)-*N*'-pyrido[1',2':1,5]pyrazolo[3,4-*d*]pyrimidin-4-ylurea;
*N*-pyrido[1',2':1,5]pyrazolo[3,4-*d*]pyrimidin-4-yl-*N*'-[4-(trifluoro methyl)phenyl]urea;
*N*-(4-fluorophenyl)-*N*'-pyrido[1',2':1,5]pyrazolo[3,4-*d*]pyrimidin-4-ylurea;
1*H*-indole-3-carbaldehyde pyrido[1',2':1,5]pyrazolo[3,4-*d*]pyrimidin-4-ylhydrazone;
or salts and solvates thereof.

7. A pharmaceutical composition comprising a compound according to any one of claims 1 to 6.

8. A compound according to any one of claims 1 to 6 for use in therapy.

9. A compound according to any one of claims 1 to 6 for use in the treatment of cancer or diabetes.

10. Use of a compound according to any one of claims 1 to 6 in the manufacture of a medicament for use in the treatment of cancer.

11. Use of a compound according to any one of claims 1 to 6 in the manufacture of a medicament for use in the treatment of diabetes.

## Patentansprüche

1. Verbindung der Formel (I): 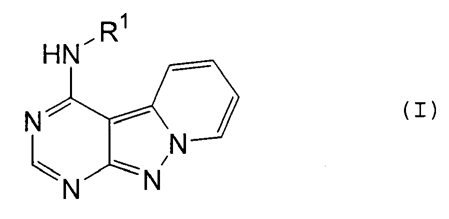 oder Salze und Solvate davon, worin
R¹ H, substituiertes Aryl, substituiertes Heteroaryl, -C(O)N(H)R² oder -N=CR³ ist, worin, wenn R¹ substituiertes Aryl oder substituiertes Heteroaryl ist, jeder Substituent unabhängig aus der Gruppe ausgewählt ist, die aus Aryl, Halogen und -OR⁴ besteht;
R² substituiertes Aryl ist, worin jeder Substituent unabhängig aus der Gruppe ausgewählt ist, die aus Cyano, Halogen, Nitro und Halogenalkyl besteht;
R³ Heteroaryl ist; und
R⁴ Aryl oder Halogenaryl ist.

2. Verbindung gemäß Anspruch 1, worin R¹ H ist.

3. Verbindung gemäß Anspruch 1, worin R¹ substituiertes Aryl oder substituiertes Heteroaryl ist, worin jeder Substituent unabhängig aus der Gruppe ausgewählt ist, die aus Aryl, Halogen und -OR⁴ besteht und R⁴ Aryl oder Halogenaryl ist.

4. Verbindung gemäß Anspruch 1, worin R¹ -C(O)N(H)R² ist und R² substituiertes Aryl ist, worin jeder Substituent unabhängig aus der Gruppe ausgewählt ist, die aus Cyano, Halogen, Nitro und Halogenalkyl besteht.

5. Verbindung gemäß Anspruch 1, worin die Verbindung ausgewählt ist aus: 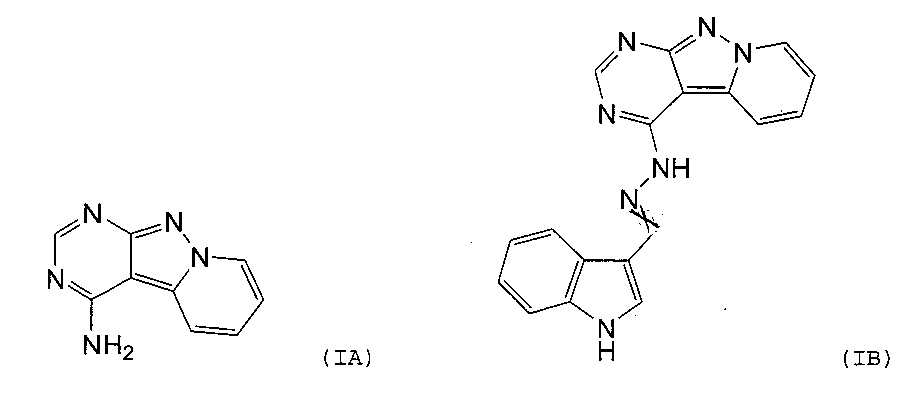 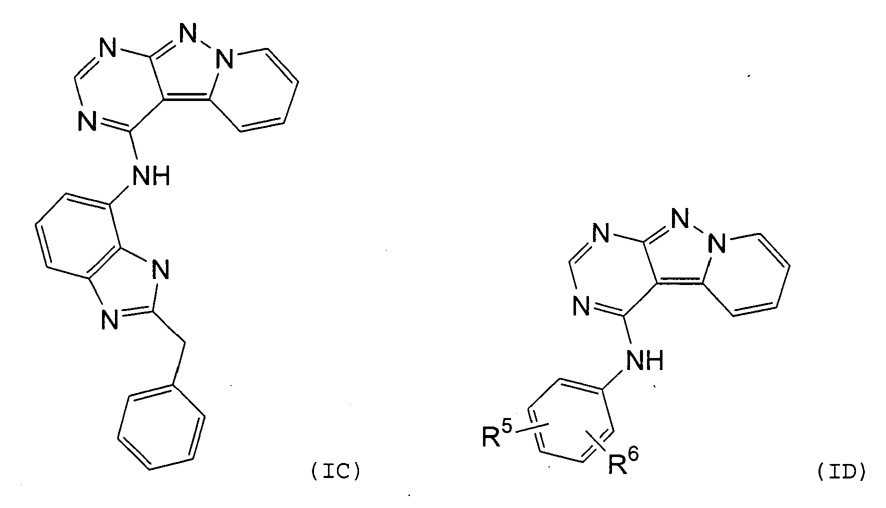 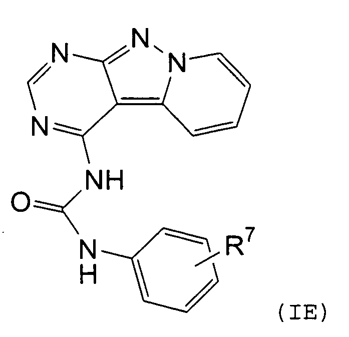 oder Salze und Solvate davon,
worin R⁵ und R⁶ unabhängig aus der Gruppe ausgewählt sind, die aus Wasserstoff, Halogen und -OR⁴ besteht;
R⁴ Aryl oder Halogenaryl ist; und
R⁷ Cyano, Halogen, Nitro und Halogenalkyl ist.

6. Verbindung, die ausgewählt ist aus:
Pyrido[1',2':1,5]pyrazolo[3,4-d]pyrimidin-4-amin,
N-(3-Chlor-4-fluorphenyl)pyrido[1',2':1,5]pyrazolo-[3,4-d]pyrimidin-4-amin,
N-(3-Chlor-4-{[(3-fluorphenyl)methyl]oxy}phenyl)-pyrido[1',2':1,5]pyrazolo[3,4-d]pyrimidin-4-amin,
N-{3-Chlor-4-[(phenylmethyl)oxy]phenyl}pyrido-[1',2':1,5]pyrazolo[3,4-d]pyrimidin-4-amin,
N-[2-(Phenylmethyl)-1H-benzimidazol-5-yl]pyrido-[1',2':1,5]pyrazolo-[3,4-d]pyrimidin-4-amin,
N-(3-Cyanophenyl)-N'-pyrido[1',2':1,5]pyrazolo-[3,4-d]pyrimidin-4-ylharnstoff,
N-Pyrido[1',2':1,5]pyrazolo[3,4-d]pyrimidin-4-yl-N'-[3-(trifluormethyl)phenyl]harnstoff,
N-(3-Nitrophenyl)-N'-pyrido[1',2':1,5]pyrazolo-[3,4-d]pyrimidin-4-ylharnstoff,
N-(4-Bromphenyl)-N'-pyrido[1',2':1,5]pyrazolo-[3,4-d]pyrimidin-4-ylharnstoff,
N-Pyrido[1',2':1,5]pyrazolo[3,4-d]pyrimidin-4-yl-N'-[4-(trifluormethyl)phenyl]harnstoff,
N-(4-Fluorphenyl)-N'-pyrido[1',2':1,5]pyrazolo-[3,4-d]pyrimidin-4-ylharnstoff,
1H-Indol-3-carbaldehydpyrido[1',2':1,5]pyrazolo-[3,4-d]pyrimidin-4-ylhydrazon,
oder Salze und Solvate davon.

7. Pharmazeutische Zusammensetzung, die eine Verbindung gemäß einem der Ansprüche 1 bis 6 umfaßt.

8. Verbindung gemäß einem der Ansprüche 1 bis 6 zur Verwendung in der Therapie.

9. Verbindung gemäß einem der Ansprüche 1 bis 6 zur Verwendung in der Behandlung von Krebs oder Diabetes.

10. Verwendung einer Verbindung gemäß einem der Ansprüche 1 bis 6 in der Herstellung eines Medikaments zur Verwendung in der Behandlung von Krebs.

11. Verwendung einer Verbindung gemäß einem der Ansprüche 1 bis 6 in der Herstellung eines Medikaments zur Verwendung in der Behandlung von Diabetes.

## Revendications

1. Composé de formule (I) 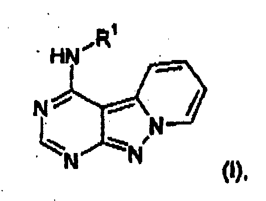 ou ses sels et solvates, dans lesquels
R¹ est un H, un aryle substitué, un hétéroaryle substitué, un -C(O)N(H)R² ou -N=CR³, dans lequel quand R¹ est un aryle substitué ou un hétéroaryle substitué, chaque substituant étant indépendamment choisi dans le groupe constitué d'aryle, halo, et -OR⁴ ;
R² est un aryle substitué, dans lequel chaque substituant est indépendamment choisi dans le groupe constitué de cyano, halo, nitro et haloalkyle ;
R³ est un hétéroaryle ; et
R⁴ est un aryle ou haloaryle.

2. Composé selon la revendication 1, dans lequel R¹ est un H.

3. Composé selon la revendication 1, dans lequel R¹ est un aryle substitué, ou un hétéroaryle substitué,
dans lequel chaque substituant est indépendamment choisi dans le groupe constitué d'un aryle, halo et -OR⁴, et
R⁴ est un aryle ou haloaryle.

4. Composé selon la revendication 1, dans lequel R¹ est un -C(O)N(H)R², et R² est un aryle substitué,
dans lequel chaque substituant est indépendamment choisi dans le groupe constitué de cyano, halo, nitro et haloalkyle.

5. Composé selon la revendication 1, dans lequel ledit composé est choisi parmi : 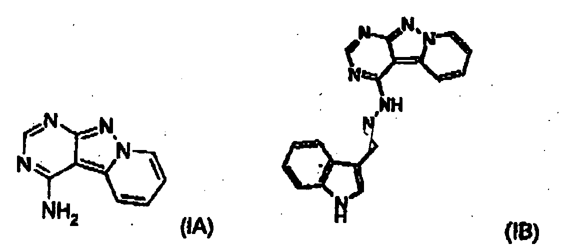 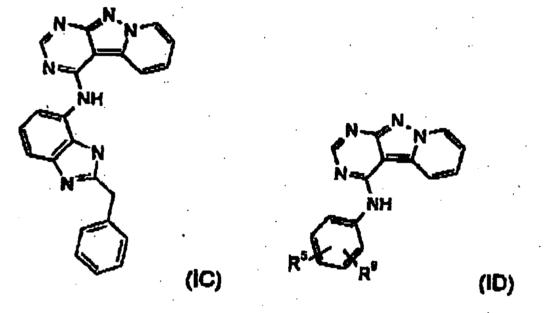 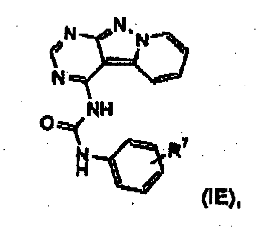 ou leurs sels ou solvates,
dans lequel R⁵ et R⁶ sont indépendamment choisis dans le groupe constitué d'hydrogène, d'halo et d'-OR⁴,
R⁴ est un aryle ou haloaryle ; et
R⁷ est un cyano, halo, nitro et haloalkyle.

6. Composé choisi parmi les éléments suivants :
pyrido [1',2' :1,5]pyrazolo[3,4-d]pyrimidin-4-amine ;
*N*-(3-chloro-4-fluorophényl)pyrido[1',2':1,5] pyrazolo[3,4-d]pyrimidin-4-amine ;
*N*-(3-chloro-4{[(3-fluorophényl)méthyl]oxy}phényl)-pyrido[1',2':1,5]pyrazolo[3,4-*d*]pyrimidin-4-amine ;
*N*-{3-chloro-4-[(phénylméthyl)oxy]phényl}pyrido [1',2' :1,5]pyrazolo-[3,4-*d*]pyrimidin-4-amine ;
*N*-[2-phénylméthyl)-1*H*-benzimidazol-5-yl]pyrido[1',2':1,5]pyrazolo-[3,4-d]pyrimidin-4-amine ;
*N*-(3-cyanophényl)-*N*'-pyrido[1',2':1,5]pyrazolo [3,4-*d*]pyrimidin-4-ylurée ;
*N*-pyrido[1',2':1,5]pyrazolo[3,4-*d*]pyrimidin-4-yl-*N'*-[3-(trifluorométhyl)phényl]urée ;
*N*-(3-nitrophényl)-*N'*-pyrido[1',2':1,5]pyrazolo [3,4-*d*]pyrimidin-4-ylurée ;
*N*-(4-bromophényl)-*N'*-pyrido[1',2':1,5]pyrazolo [3,4-*d*]pyrimidin-4-ylurée ;
*N*-pyrido[1',2':1,5]pyrazolo[3,4-*d*]pyrimidin-4-yl-*N'*-[4-(trifluorométhyl)phényl]urée ;
*N*-(4-fluorophényl)-*N'*-pyrido[1',2':1,5]pyrazolo [3,4-*d*]pyrimidin-4-ylurée ;
1*H*-indole-3-carbaldéhyde pyrido[1',2':1,5]pyrazolo [3,4-*d*]pyrimidin-4-ylhydrazone ;
ou des sels et solvates de ceux-ci.

7. Composition pharmaceutique comprenant un composé selon l'une quelconque des revendications 1 à 6.

8. Composé selon l'une quelconque des revendications 1 à 6, à utiliser en thérapie.

9. Composé selon l'une quelconque des revendications 1 à 6, à utiliser dans le traitement du cancer ou du diabète.

10. Utilisation d'un composé selon l'une quelconque des revendications 1 à 6, dans la fabrication d'un médicament à utiliser dans le traitement du cancer.

11. Utilisation d'un composé selon l'une quelconque des revendications 1 à 6, dans la fabrication d'un médicament à utiliser dans le traitement du diabète.
